Europäisches Patentamt

(19) European Patent Office

Office européen des brevets

(11) Publication number : **0 242 892 B1**

(12) **EUROPEAN PATENT SPECIFICATION**

(45) Date of publication of patent specification :
21.11.91 Bulletin 91/47

(51) Int. Cl.⁵ : **C12N 15/70, C05F 11/08, A01N 63/00**

(21) Application number : **87200348.8**

(22) Date of filing : **26.02.87**

(54) **Process for activating rhizobium nodulation promoters.**

(30) Priority : 28.02.86 NL 8600512

(43) Date of publication of application :
28.10.87 Bulletin 87/44

(45) Publication of the grant of the patent :
21.11.91 Bulletin 91/47

(84) Designated Contracting States :
AT BE CH DE ES FR GB GR IT LI LU NL SE

(56) References cited :
EP-A- 0 064 720
EP-A- 0 108 508
EP-A- 0 164 992
EP-A- 0 167 192
DE-C- 749 153
CHEMICAL ABSTRACTS, vol. 106, no. 19, 11th
May 1987, page 173, abstract no. 150549g,
Columbus, Ohio, US; H.P. SPAINK et al.:
"Regulation of the promoters in the nodu-
lation region of the symbiosis plasmid pRL1JI
of Rhizobium leguminosarum", & MOL.
GENET. PLANT-MICROBE INTERACT., PROC.
INT. SYMP., 3rd 1986 (Pub. 1987) 244-6
CHEMICAL ABSTRACTS, vol. 106, no. 19, 11th
May 1987, page 159, abstract no. 150419q,
Columbus, Ohio, US; S.A.J. ZAAT et al.:
"Naringenin induces the nodABC promotor of
Rhizobium leguminosarum as well as Tsr fac-
tor production", & MOL. GENET. PLANT-
MICROBE INTERACT., PROC. INT. SYMP., 3rd
1986 (Pub. 1987), 247-9
PROC. NATL. ACAD. SCI. USA, vol. 83, Decem-
ber 1986, pages 9581-9585; J. SCHMIDT et al.:
"Expression of the nodulation gene nodA in
Rhizobium meliloti and localization of the
gene product in the cytosol"
NATURE, vol. 324, 6th November 1986, pages
90-92; J.L. FIRMIN et al.: "Flavonoid activation
of nodulation genes in Rhizobium reversed by
other compounds present in plants"

(73) Proprietor : **Rijksuniversiteit Leiden**
**Rapenburg 73**
**Leiden (NL)**

(72) Inventor : **Wijffelman, Carolus A.**
**Johan de Wittstraat 28**
**NL-2334 AR Leiden (NL)**
Inventor : **Spaink, Herman Pieter**
**Badhuisweg 23**
**NL-2587 CB Scheveningen (NL)**
Inventor : **Okker, Robert Jan Hendrik**
**Leidsestraatweg 14**
**NL-2341 GR Oegstgeest (NL)**
Inventor : **van Brussel, Antonius Albertus N.**
**Boerhaavelaan 66**
**NL-2334 ES Leiden (NL)**
Inventor : **Zaat, Sebastianus A. J.**
**Marislaan 9**
**NL-2316 XV Leiden (NL)**
Inventor : **Lugtenberg, Egbertus J. J.**
**Gebr. van Eyckstraat 7**
**NL-3443 VG Woerden (NL)**

(74) Representative : **Huygens, Arthur Victor, Dr. et**
**al**
**c/o Gist-Brocades N.V. Patent & Trademarks**
**Department Wateringseweg 1 PO Box 1**
**NL-2600 MA Delft (NL)**

(56) References cited :

CHEMICAL ABSTRACTS, vol. 89, no. 25, 18th December 1978, page 305, abstract no. 211927h, Columbus, Ohio, US; M. JAY et al.: "Flavonoids of dorycnium suffructicosum and tetragonolobus siliquosus (Leguminosae)", & PHYTOCHEMISTRY 1978, 17(7), 1196-8

CHEMICAL ABSTRACTS, vol. 104, no. 13, 31st March 1986, page 424, abstract no. 106305j, Columbus, Ohio, US; P.M. PORTER et al.: "Phenolic acids and flavonoids in soybean root and leaf extracts", & ENVIRON. EXP. BOT. 1986, 26(1), 65-73

CHEMICAL ABSTRACTS, vol. 89, no. 13, 25th September 1978, page 428, abstract no. 103708d, Columbus, Ohio, US; N. ISHIKURA et a.: "Paper chromatographic survey of anthocyanins in leguminosae. III. Identification and distribution pattern of anthocynins in twenty-two legumes", & BOT. MAG. 1978, 91(1021), 25-30

MOLECULAR AND GENERAL GENETICS, vol. 201, 1985, pages 426-432, Springer Verlag; R.W. INNES et al.: "Plant factors induce expression of nodulation and host-range genes in Rhizobium trifolii"

PROC. NATL. ACAD. SCI. USA, vol. 82, October 1985, pages 6609-6613; J.T. MULLIGAN et al.: "Induction of Rhizobium meliloti nodC expression by plant exudate requires nodD"

THE EMBO JOURNAL, vol. 4, no. 13A, 1985, pages 3369-3373, IRL Press Ltd, Oxford, GB; L. ROSSEN et al.: "The nodD gene of Rhizobium leguminosarum is autoregulatory and in the presence of plant exudate induces the nodA,B,C genes"

CHEMICAL ABSTRACTS, vol. 67, no. 13, 25th September 1967, page 5916, abstract no. 63148h, Columbus, Ohio, US; J.A.E. MOLINA et al.: "The effect of antimetabolites on nodulation and growth of leguminous plants", & CAN. J. MICROBIOL. 13(7), 819-27, (1967)

JOURNAL OF PLANT PHYSIOLOGY, vol. 122, no. 1, January 1986, pages 25-40, Gustav Fischer, Stuttgart-New York; H.C.J. CANTER CREMERS et al.: "Sym plasmid and chromosomal gene products of Rhizobium trifolii elicit developmental responses on various legume roots"

NATURE, vol. 318, no. 6047, December-January 1985/1986, pages 624-629, London, GB; S.E. STACHEL et al.: "Identification of the signal molecules produced by wounded plant cells that activate T-DNA transfer in Agrobacterium tumefaciens"

CHEMICAL ABSTRACTS, vol. 102, no. 13, 1st April 1985, page 422, abstract no. 110120w, Columbus, Ohio, US; T.V. BHUVANESARI et al.: "Root hair deformation in the white clover/Rhizobium trifolii symbiosis", & PHYSIOL. PLANT. 1985, 63(1), 25-34

JOURNAL OF BACTERIOLOGY, vol. 165, no. 2, February 1986, pages 517-522, American Society for Microbiology, US; A.A.N. VAN BRUSSEL et al.: "Role of plant root exudate and sym plasmid-localized nodulation genes in the synthesis by Rhizobium leguminosarum of Tsr factor, which causes thick and short roots on common vetch"

## Description

This invention relates to a process for activating Rhizobium nodulation promoters.

Bacteria of the genus Rhizobium, which are able to live in symbiosis with leguminous plants, invade the roots, where they induce the formation of nodules in which they fix atmospheric nitrogen. In Rhizobia, which include Rhizobium leguminosarum, with pea and vetch as their hosts, R.trifolii, with clovers as their hosts, and R.meliloti, with alfalfa as their host, many genes involved in nodulation and N$_2$-fixation are localized on large plasmids, which have been designated Sym(biosis) plasmids. The genetic organization and regulation of the Sym plasmid-localized nodulation (nod) genes has already been investigated. Until now ten nod genes organized in four operons have been identified in R.leguminosarum. These nod genes can be divided in common and host-specific genes. The genetic organization of the common nod genes nod A,B,C,D,I,J, of the other Rhizobia is highly homologous with that described for R.leguminosarum, whereas the genetic organization of the host-specific genes is more divergent. Evidence is accumulating that the expression of Sym plasmid-localized nod genes in Rhizobia is regulated as a single regulon.

Whereas the nodD gene of the Rhizobia is transcribed constitutively, several other nod genes appear not to be transcribed detectably in the laboratory media tested till now. However, recent results indicate that several nod operons are induced when bacteria are incubated in the presence of root exudates of legumes (Nitrogen Fixation Research Progress pages 79-85, 87-93, 95-100,(1985)). Induction also requires a functional nodD product.

In order to improve the nitrogen fixation Rhizobium bacteria are contacted in practice sometimes on purpose with seed or plant (inoculation). These bacteria may be selected on the basis of good qualities present or they may have obtained these through intentional engineering. With inoculation by Rhizobia cultured for that purpose the problem arises that cultured strains often appear to be no match for Rhizobia already present in the ground. When the improved Rhizobia are cultured in the presence of inducer present in exudate, they are better equiped for the competition, because the nod genes have already been switched on. Therefore, it is very important to find the inducer present in exudate, which may also be used in many other applications.

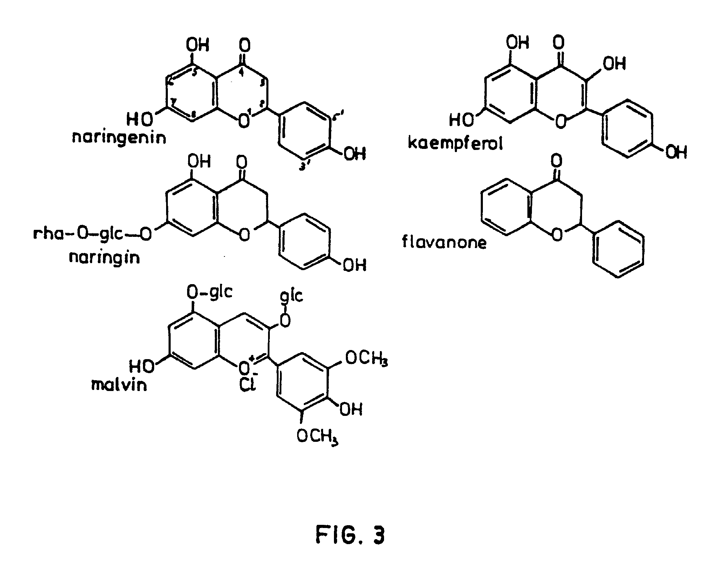

Now we have found that flavan derivatives having oxygen-containing groups in the positions 5, 7 and 4', and only one keto or optionally substituted enol group in the position 3 or 4, may be used instead of plant exudate for inducing exudate-inducible nod operons of Rhizobia. The preferred compound for inducing exudate-inducible nod operons of R.leguminosarum and R.trifolii is naringenin (see fig. 3). The preferred compound for inducing exudate-inducible nod operons of R.meliloti is malvin (see fig. 3). Both naringenin and malvin are plant metabolites.

Experiments which led to this finding are as follows.

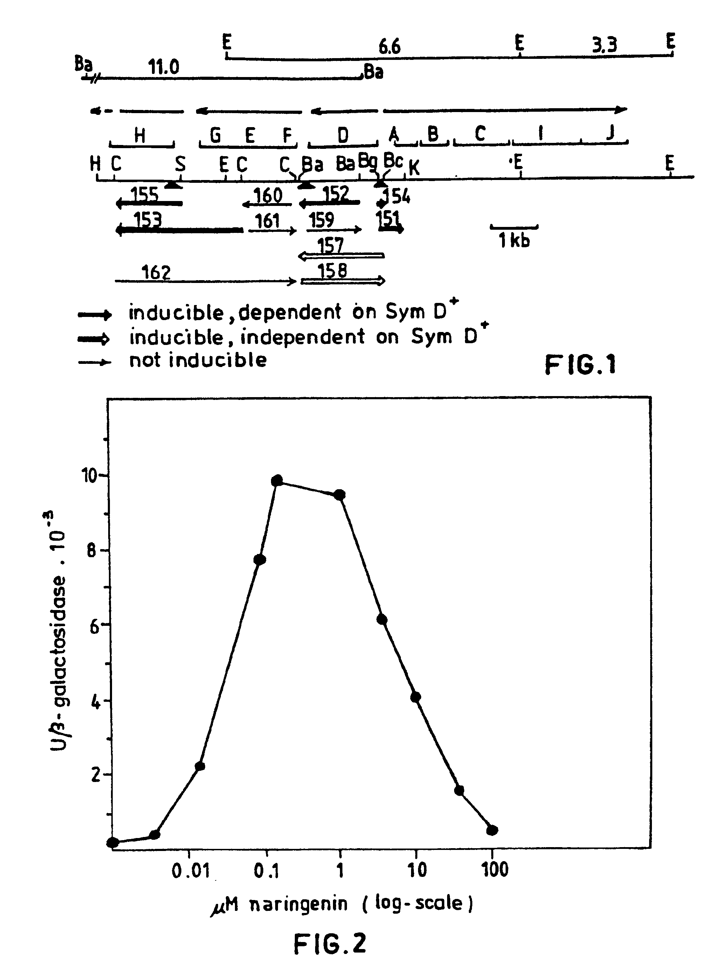

Fig. 1 represents the nodulation region of the R.leguminosarum Sym plasmid pRL1JI. The position of the known nod genes or open reading frames are indicated, together with the operon structure and direction of transcription. The EcoRI 9.9 kb double fragment and the BamHI 11.0 kb fragment of the nod region were cloned. Together these fragments contain all identified Sym plasmid-localized nod genes and their promoters. Subclones from these fragments, some of which are indicated, were isolated in the transcriptional fusion vector pMP150. The vector pMP150 is a 18 kb derivative of the broad host range, mobilizable, plasmid RSF1010 of the IncQ incompatibility group. The vector contains resistance markers for streptomycin and chloramphenicol, a multiple cloning sequence, the Shine-Dalgarno sequence from the E.coli chloramphenicol acetyl transferase gene and the structural gene lacZ of E.coli β-galactosidase without its promoter. Except for a weak background activity, the lacZ gene is only expressed if a promoter sequence is cloned in the correct orientation in the multiple cloning site. The direction of the arrows in fig. 1 indicates the orientation of the subcloned fragments towards lacZ of pMP150. Plasmids with subcloned fragments were mobilized from E.coli to Rhizobium using either pRK 2013 or R772 as a helper plasmid and with rifampicin (20 μg.ml⁻¹), streptomycin (1 mg.ml⁻¹) and chloramphenicol (10 μg.ml⁻¹) as selection markers. Rhizobium acceptor strains were the isogenic strains LPR5045 (without Sym plasmid), LPR5560 (with Tn5 marked R.leguminosarum Sym plasmid pRL1JI) and LPR5561 (with pRL1JI nodD::Tn5). Strain LPR5560 shows symbiosis properties identical ot other R.leguminosarum strains. The subcloned fragments were tested for inducibility of β-galactosidase by root exudates of Vicia hirsuta or V. sativa, flavan derivatives and other substances. The dependence of expression on the presence of a Sym plasmid with an intact nodD gene and/or inducer (exudate or flavan derivative of the invention) is indicated in fig. 1. The position of the exudate and nodD protein dependent promoters is also indicated (by triangles). Restriction sites are only indicated when relevant as borders of subclones used. H, HindIII; C, ClaI; C', ClaI dam methylated; S, SalI; E, EcoRI; Bc, BclI; Ba, BamHI; Bg; BglII; K, KpnI.

The following table relates to the test of the resulting strains for inducibility by root exudates of Vicia hirsuta or V.sativa.

TABLE

Dependence of induction of nod promoters from exudate and nodD product provided by a Sym plasmid.

| Expression plasmid and tested promoter | Presence (+) or absence (-) of Sym plasmid pRL1JI encoded nodD product | Units β-galactosidase | |
| --- | --- | --- | --- |
| | | - exudate | +exudate |
| Rhizobium LPR5560, LPR5045 (without expression vector) | + | 15 | 15 |
| | - | 15 | 15 |
| pMP150 (without promoter) | + | 50 | 50 |
| pMP151 (p.nodA),pMP152(p.nodF) pMP153 (p.nodH),pMP154(p.nodA) pMP155 (p.nodH) | - | 150-500 | 150-500 |
| | + | 150-500 | $4-14 \times 10^3$ |
| pMP157 (p.nodF),pMP158(p.nodA) | - | 150-350 | $10-14 \times 10^3$ |
| | + | 150-350 | $10-14 \times 10^3$ |
| pMP159, pMP160, pMP161, pMP162, (no promoter present or not present in correct orientation) | + | 100-600 | 100-600 |

Exudates were prepared by growing Vicia hirsuta seedlings during 2 days with roots submerged in Jensen medium. One axenic Vicia plant was used for 10 ml exudate. The plants were removed, and 20% v/v B⁻ medium and 1 mg.ml⁻¹ streptomycin were added. These B⁻-exudates (pH 6.0) were inoculated to an $A_{660\,nm}$ value of 0.03 with Rhizobium cells grown in B⁻ medium. After the standard induction period of 18 hours at 28°C, an $A_{660\,nm}$ value of the suspension of 0.06 was reached. The bacteria were lysed with toluene and the activity of β-galactosidase was determined according to the method of Miller. Exudates of V.sativa, prepared in the same way, gave comparable results.

From the above data three root exudate-inducible nod promoters could be identified in R.leguminosarum which were designated as promoter p.nodA, p.nodF and p.nodH. For p.nodA this was already known. Similar results were found for the R.trifolii promoters p.nodA and p.hsnA. The latter confirms the results of Innes et al (Molec. gen. Genet. 201, 426-432; 1985). Similar results were also found for the R.meliloti nodA promoter. The latter promoter was shown to be activated as well by soak water from Medicago seeds.

The induction of all tested exudate-inducible promoters of R.leguminosarum, R.trifolii and R.meliloti is dependent on the presence of a functional nodD product, which in the present experiments was either provided by the Sym plasmid pRL1JI or by the cloned fragment when it contained an intact nodD gene.

Since the isolation of the inducer from plant exudate appeared laborious and time-consuming, another way was chosen to identify a compound with inducing activity. A number of plant products was tested for inducing activity. Some of these plant products are: phaseollin, 3,4,5-trimethylbenzaldehyde, acetosyringon, kaempferol, flavanone, naringin, malvin and naringenin. Rhizobium strain LPR5045 (without Sym plasmid) containing pMP157 (p.nodF) was used for this test.

Among these plant products the compounds naringenin, naringin and malvin showed the inducing activity of root exudate. All the other compounds tested showed no activity. Also the tested compound acetosyringon and structurally related compounds, known to induce the vir-operons of Agrobacterium tumefaciens, appear to be not active here.

Induction by naringenin and exudate of V.hirsuta was followed during one hour. Extrapolation of the results showed that β-galactosidase synthesis started 5 minutes after addition of naringenin or exudate. Significant induction by naringenin (using pMP154 or pMP157 and a 18 h. incubation period) could already be observed at 1.0 nM. Induction levels increased linearly with naringenin concentrations up to 20 nM. Half-maximal and maximal induction (with pMP157) were observed at 30 and 100 nM, respectively. Growth of Rhizobium was adversely affected above 2000 nM and the level of induction decreased (fig. 2). Naringenin induced all three exudate-inducible R.leguminosarum nod promoters as well as the R.trifolii p.nodA and p.hsnA in a similar way as V.hirsuta exudate, Naringin and malvin induced these promoters at concentrations at least 1000-fold higher. The compound malvin on the other hand induced the exudate-inducible R.meliloti nodA promoter in a similar way as V.hirsuta exudate.

## Claims

1. A process for activating nodulation promoters of Rhizobium bacteria by incubating said Rhizobium bacteria in the presence of a product having inducing activity, characterized in that the Rhizobium bacteria are incubated in the presence of at least one flavan derivative having oxygen-containing groups in the positions 5, 7 and 4', and only one keto or optionally substituted enol group in the position 3 or 4.

2. A process according to claim 1, characterized in that the Rhizobium is R.leguminosarum or R.trifolii, and that the compound naringenin is used as said flavan derivative.

3. A process according to claim 2, characterized in that the naringenin concentration is 1-2000 nM.

4. A process according to claim 3, characterized in that the naringenin concentration is 100 nM.

5. A process according to claim 1, characterized in that the Rhizobium is R.meliloti, and that the compound malvin is used as said flavan derivative.

6. A process for improving the ability to fix nitrogen of germinating seed or a growing plant sprouted therefrom, wherein said seed or said plant is contacted with Rhizobium bacteria, characterized in that said seed or said plant is moreover contacted with at least one flavan derivative having oxygen-containing groups in the positions 5, 7 and 4', and only one keto or optionally substituted enol group in the position 3 or 4.

7. A process according to claim 6, characterized in that the Rhizobium is R.leguminosarum or R.trifolii, and that the compound naringenin is used as said flavan derivative.

8. A process according to claim 7, characterized in that the naringenin concentration is 1-2000 nM.

9. A process according to claim 8, characterized in that the naringenin concentration is 100 nM.

10. A process according to claim 6, characterized in that the Rhizobium is R.meliloti, and that the compound malvin is used as said flavan derivative.

## Revendications

1. Procédé pour activer les promoteurs de nodulation de bactéries Rhizobium par incubation de ces bactéries Rhizobium en présence d'un produit ayant une activité d'induction, caractérisé en ce que les bactéries Rhizobium sont incubées en présence d'au moins un dérivé de flavane portant des radicaux contenant de l'oxygène aux positions 5, 7 et 4' et un seul radical céto ou énol facultativement substitué à la position 3 ou 4.

2. Procédé suivant la revendication 1, caractérisé en ce que le Rhizobium est R.leguminosarum ou R.trifolii et la naringénine est utilisée comme dérivé du flavane.

3. Procédé suivant la revendication 2, caractérisé en ce que la concentration en naringénine est 1 à 2000 nM.

4. Procédé suivant la revendication 3, caractérisé en ce que la concentration en naringénine est 100 nM.

5. Procédé suivant la revendication 1, caractérisé en ce que le Rhizobium est R.meliloti et la malvine est utilisée comme dérivé du flavane.

6. Procédé pour améliorer l'aptitude à fixer l'azote d'une semence en germination ou d'une plante en croissance qui en est issue, dans lequel la semence ou la plante est mise en contact avec des bactéries Rhizobium, caractérisé en ce que la semence ou la plante est en outre mise en contact avec au moins un dérivé de flavane portant des radicaux contenant de l'oxygène aux positions 5, 7 et 4' et un seul radical céto ou énol facultativement substitué à la position 3 ou 4.

7. Procédé suivant la revendication 6, caractérisé en ce que le Rhizobium est R.leguminosarum ou R.trifolii et la naringénine est utilisée comme dérivé du flavane.

8. Procédé suivant la revendication 7, caractérisé en ce que la concentration en naringénine est 1 à 2000 nM.

9. Procédé suivant la revendication 8, caractérisé en ce que la concentration en naringénine est 100 nM.

10. Procédé suivant la revendication 6, caractérisé en ce que le Rhizobium est R.meliloti et la malvine est utilisée comme dérivé du flavane.


## Patentansprüche

1. Verfahren zum Aktivieren von Knöllchenbildungspromotoren von Rhizobium-Bakterien durch Inkubieren der genannten Rhizobium-Bakterien in Gegenwart eines Produktes mit Induzierungsaktivität, dadurch gekennzeichnet, dass die Rhizobium-Bakterien in Gegenwart von mindestens einem Flavanderivat mit sauerstoffhaltigen Gruppen in den Stellungen 5, 7 und 4' und nur einer Keto- oder gegebenenfalls substituierten Enolgruppe in der Stellung 3 oder 4 inkubiert werden.

2. Verfahren nach Anspruch 1, dadurch gekennzeichnet, dass das Rhizobium R. leguminosarum oder R. trifolii ist und dass die Verbindung Naringenin als genanntes Flavanderivat verwendet wird.

3. Verfahren nach Anspruch 2, dadurch gekennzeichnet, dass die Naringeninkonzentration 1 bis 2000 nMol/l beträgt.

4. Verfahren nach Anspruch 3, dadurch gekennzeichnet, dass die Naringeninkonzentration 100 nMol/l beträgt.

5. Verfahren nach Anspruch 1, dadurch gekennzeichnet, dass das Rhizobium R. meliloti ist und dass die Verbindung Malvin als genanntes Flavanderivat verwendet wird.

6. Verfahren zur Verbesserung der Fähigkeit zum Fixieren von Stickstoff von keimendem Samen oder einer daraus aufgegangenen wachsenden Pflanze, wobei der genannte Samen oder die genannte Pflanze mit Rhizobium-Bakterien in Berührung gebracht wird, dadurch gekennzeichnet, dass der genannte Samen oder die genannte Pflanze überdies mit mindestens einem Flavanderivat mit sauerstoffhaltigen Gruppen in den Stellungen 5, 7 und 4' und nur einer Keto- oder gegebenenfalls substituierten Enolgruppe in der Stellung 3 oder 4 in Berührung gebracht wird.

7. Verfahren nach Anspruch 6, dadurch gekennzeichnet, dass das Rhizobium R. leguminosarum oder R. trifolii ist und dass die Verbindung Naringenin als genanntes Flavanderivat verwendet wird.

8. Verfahren nach Anspruch 7, dadurch gekennzeichnet, dass die Naringeninkonzentration 1 bis 2000 nMol/l beträgt.

9. Verfahren nach Anspruch 8, dadurch gekennzeichnet, dass die Naringeninkonzentration 100 nMol/l beträgt.

10. Verfahren nach Anspruch 6, dadurch gekennzeichnet, dass das Rhizobium R. meliloti ist und dass die Verbindung Malvin als genanntes Flavanderivat verwendet wird.

→ inducible, dependent on Sym D⁺
⇒ inducible, independent on Sym D⁺
→ not inducible

FIG.1

FIG.2

FIG. 3